(19) Europäisches Patentamt
European Patent Office
Office européen des brevets

(11) Veröffentlichungsnummer: **0 215 354 B1**

(12) **EUROPÄISCHE PATENTSCHRIFT**

(45) Veröffentlichungstag der Patentschrift: **24.04.91**

(51) Int. Cl.5: **C07D 253/06**, A61K 31/53

(21) Anmeldenummer: **86111924.6**

(22) Anmeldetag: **28.08.86**

Die Akte enthält technische Angaben, die nach dem Eingang der Anmeldung eingereicht wurden und die nicht in dieser Patentschrift enthalten sind.

# (54) Substituierte 2-Phenyl-hexahydro-1,2,4-triazin-3,5-dione, Verfahren zu ihrer Herstellung, sie enthaltende Mittel und ihre Verwendung.

(30) Priorität: **07.09.85 DE 3531919**

(43) Veröffentlichungstag der Anmeldung:
**25.03.87 Patentblatt 87/13**

(45) Bekanntmachung des Hinweises auf die Patenterteilung:
**24.04.91 Patentblatt 91/17**

(84) Benannte Vertragsstaaten:
**AT BE CH DE FR GB IT LI NL**

(56) Entgegenhaltungen:
**EP-A- 0 058 534**
**EP-A- 0 170 316**
**DE-A- 2 423 972**
**EP-A- 0 154 885**
**DE-A- 2 149 645**
**DE-A- 2 722 537**

(73) Patentinhaber: **HOECHST AKTIENGESELLSCHAFT**
**Postfach 80 03 20**
**W-6230 Frankfurt am Main 80(DE)**

(72) Erfinder: **Rösner, Manfred, Dr.**
**Unter den Buchen 7**
**W-6239 Eppstein/Taunus(DE)**
Erfinder: **Raether, Wolfgang, Dr.**
**Falkensteinstrasse 6**
**W-6072 Dreieich(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Coccidiostatisch wirksame 1,2,4-Triazin-3,5-(2H,4H)-dione und ihre Herstellung sind u.a. nach DE-OS 24 23 972, DE-OS 21 49 645 und DE-OS 27 22 537 = US 41 98 407 bekannt. In EP-A-58 534 sind Hexahydro-1,2,4-triazin-3,5-dione mit einem Benzyl- oder Thienylmethylsubstituenten in 2-Stellung beschrieben. Diese besitzen jedoch unzureichende Wirksamkeiten.

Gegenstand der Erfindung sind daher 2-Phenyl-hexahydro-1,2,4-triazin-3,5-dione der Formel I oder deren Salze

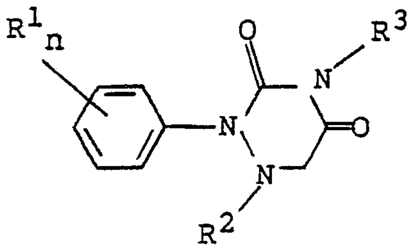

(I)

worin

n

eins, zwei oder drei

und die einzelnen Substituenten

$R^1$

unabhängig voneinander

Phenoxy-, Phenylthio-, Phenylsulfinyl- oder Phenylsulfonylrest, der ein- oder zweifach durch F, Cl, Br, $CF_3$, Alkyl, Alkoxy, Alkylthio, Alkylsulfinyl oder Alkylsulfonyl mit jeweils 1 bis 6 C-Atomen im Alkylrest substituiert sein kann,

$R^2$

Wasserstoff, geradkettiges oder verzweigtes Alkyl oder Alkanoyl mit jeweils 1 bis 4 C-Atomen, Cycloalkyl mit 3 bis 7 C-Atomen, Benzyl oder Benzoyl, die unsubstituiert oder durch F, Cl, Br oder ($C_1$-$C_4$)-Alkyl substituiert sind;

$R^3$

Wasserstoff, geradkettiges oder verzweigtes Alkyl mit 1 bis 12 C-Atomen, Cycloalkyl mit 3 bis 7 C-Atomen oder unsubstituiertes oder durch F, Cl, Br oder ($C_1$-$C_4$)-Alkyl substituiertes Benzyl;

unter der Voraussetzung, daß mindestens einer der Reste $R^2$ und $R^3$ ungleich Wasserstoff ist,

sowie deren Alkali-, Erdalkali- oder Ammoniumsalze, sofern $R^3$ Wasserstoff bedeutet.

Bevorzugt sind solche Verbindungen der Formel I, in der einer oder mehrere Substituenten folgende Bedeutungen besitzen:

n

2 oder 3

$R^1$

unabhängig voneinander

Phenoxyrest, der ein- oder zweifach durch Cl, $CF_3$, ($C_1$-$C_4$)-Alkyl, ($C_1$-$C_4$)-Alkylthio, ($C_1$-$C_4$)-Alkylsulfinyl oder ($C_1$-$C_4$)-Alkylsulfonyl substituiert sein kann,

$R^2$ = ($C_1$-$C_4$)-Akyl, ($C_1$-$C_4$)-Alkanoyl oder Benzyl;

$R^3$ = H, ($C_1$-$C_4$)-Alkyl oder Benzyl,

und worin vorzugsweise der Phenylrest in Formel I in 3,5-Stellung disubstituiert oder 3,4,5-Stellung trisubstituiert ist;

Die Erfindung betrifft weiterhin ein Verfahren zur Herstellung von substituierten Hexahydro-1,2,4-triazin-3,5-dionen der Formel I, dadurch gekennzeichnet, daß man ein substituiertes Hexahydro-1,2,4-triazin3,5-dion der Formel II,

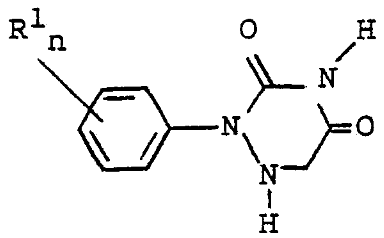

(II),

worin n und $R^1$ die zu Formel I gegebenen Bedeutungen haben, alkyliert oder acyliert.

Die Verbindungen der Formel II werden durch selektive Hydrierung der C-N-Doppelbindung in Verbindungen der Formel III erhalten

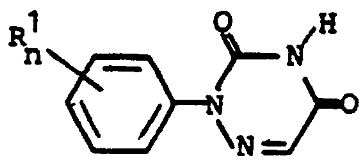

(III)

worin n und $R^1$ die zur Formel I gegebenen Bedeutungen haben.

Die Reduktion der Verbindungen der Formel III erfolgt nach allgemein bekannten Verfahren. Sie kann je nach den Gegebenheiten der Verbindung der Formel II durch katalytisch angeregten Wasserstoff mit Katalysatoren wie Raney-Nickel, Platin, Palladium, Platin-(IV)-oxid oder durch chemische Reduktion mit Metallen, Metallsalzen, Metallcarbonylen oder komplexen Hydriden erfolgen. Beispielhaft seien genannt Natriumamalgam in Ethanol, Lithium in Ammoniak, Zinn-(II) chlorid in Salzsäure, Eisen in Eisessig, Lithiumaluminiumhydrid, Natriumborhydrid, Natriumcyanoborhydrid. Vorzugsweise erfolgt die Reduktion mit Zink in Eisessig oder Zinn-(II) chlorid in Salzsäure, ggf. in einem inerten Lösungs- oder Verdünnungsmittel wie Methanol, Ethanol, Toluol, Aceton, Butanon, Dimethoxyethan, Tetrahydrofuran, Dioxan, Essigsäureethylester, Pyridin, Eisessig.

Man arbeitet z.B. in einem Temperaturbereich von etwa 50 °C bis 150 °C, vorzugsweise zwischen 80 °C und 120 °C oder bei der Siedetemperatur des verwendeten Lösungsmittels oder Lösungsmittelgemisches.

Die Darstellungsverfahren für Verbindungen der Formel III sind literaturbekannt.

Die Herstellung erfolgt zum Beispiel durch Diazotierung eines entsprechend substituierten Anilinderivates und Kupplung des Diazoniumsalzes mit N,N'-Bis(ethoxycarbonyl)-malonsäurediamid mit anschließender Cyclisierung, Verseifung und Decarboxylierung.

Die Darstellung von Verbindungen der Formel I, in denen $R^3$ nicht Wasserstoff bedeutet, erfolgt nach allgemein bekannten Methoden mittels einer Base wie Natriumhydrid, Natriumhydroxid oder Triethylamin und einem Alkylierungsmittel der Formel IV,

$$R^3 - X \qquad (IV)$$

worin

$R^3$

die zur Formel I gegebenen Bedeutungen mit Ausnahme von Wasserstoff hat und

X

eine Abgangsgruppe wie z.B. Cl, Br, J, Tosylat oder Mesylat bedeutet.

Die Darstellung von Verbindungen der Formel I, in denen $R^2$ nicht Wasserstoff bedeutet, erfolgt durch Umsetzung einer Verbindung der Formel II mit einem Alkylierungs- oder Acylierungsmittel der Formel V

$$R^2 - Y \qquad (V)$$

worin

$R^2$

die zur Formel I gegebenen Bedeutungen mit Ausnahme von Wasserstoff hat und

Y

eine Abgangsgruppe wie Cl, Br, J und im Falle einer Alkylierung auch Tosylat oder Mesylat, im Falle einer Acylierung auch die dem Acylrest zugrunde liegende Carbonsäure oder deren Anhydrid bedeutet.

Die Alkylierung mit einer Verbindung der Formel V erfolgt ohne oder zweckmäßigerweise mit einem inerten Lösungsmittel wie z.B Toluol, Xylol, Dimethylformamid, Dimethylsulfoxid, Dimethylsulfon, N-Methylpyrrolidon oder deren Gemischen durch Erhitzen mit dem Alkylierungsmittel auf z.B. 100-250° C bei Normaldruck oder im Autoklaven. Die Reaktionszeiten liegen dabei z. B. zwischen 2 und 50 h.

Die Acylierung mit einer Verbindung der Formel V erfolgt mit oder ohne Lösungs- oder Verdünnungsmittel, vorzugsweise in einem Überschuß des Acylierungsmittels mit oder ohne Katalysator. Als solche kommen z. B. konzentrierte Schwefelsäure, aber auch Säurechloride wie z.B. Acetylchlorid, Propionylchlorid, Chlorameisensäureethylester, Thionylchlorid oder Phosphoroxidchlorid in Betracht, die das vorgesehene Acylierungsmittel in geeigneter Weise aktivieren. Die Reaktion kann z.B. in einem Temperaturbereich von -20° bis +150° C, vorzugsweise bei 0 bis 80° C durchgeführt werden. Je nach den angewandten Reaktionsbedingungen können die Reaktionszeiten zwischen 30 Minuten und 24 Stunden liegen.

Wenn auf die vorstehend beschriebene Weise Verbindungen der Formel I erhalten werden, in denen $R^3$ Wasserstoff bedeutet, so können diese nach allgemein bekannten Methoden mittels einer Base wie Natriumhydrid, Natriumhydroxid oder Triethylamin und einem Alkylierungsmittel der Formel IV in Verbindungen der Formel I mit $R^3$ ungleich Wasserstoff umgesetzt werden.

Im Falle der Synthese einer Verbindung der Formel I, in der $R^2$ und $R^3$ den gleichen Alkylrest bedeuten, kann wahlweise eine stufenweise oder eine gleichzeitige Einführung der beiden gleichen Alkylreste mittels einer Kombination der vorstehend beschriebenen Reaktionsbedingungen durchgeführt werden. Z. B. arbeitet man dann in einem inerten Lösungsmittel wie N-Methylpyrrolidon mit einem Überschuß des Alkylierungsmittels in Anwesenheit einer Base wie Natriumhydrid bei 100°-250° C und bei Normaldruck oder im Autoklaven.

Die Verbindungen der Formel I, in denen $R^3$ Wasserstoff bedeutet, können durch Zugabe von zweckmäßig einem Muläquivalent Alkali, Erdalkali, Ammoniak oder Aminen in die entsprechenden Salze übergeführt werden.

Bevorzugt verwendet man dafür Natriumhydroxid, Natriummethylat, Natriumhydrid, Kaliumhydroxid, Calciumhydroxid, Calciumhydrid, Ammoniak, Alkylamine, Alkylenderivate oder Alkanolamine wie z.B. Ethanolamin.

Die erfindungsgemäßen Verbindungen der Formel I sind Chemotherapeutika, die gegen Protozoenerkrankungen insbesondere als Coccidiostatika verwendet werden können.

Die Coccidiose verursacht in der Geflügelhaltung Mortalität und damit große wirtschaftliche Verluste. Deshalb sind prophylaktische und therapeutische Maßnahmen notwendig. Im Vordergrund steht die Prophylaxe, insbesondere die Verabreichung von Coccidiostatika im Futter, die den Ausbruch einer Coccidiose verhindert. Daneben können diese Mittel auch therapeutisch bei einer bereits vorliegenden Coccidiose eingesetzt werden.

Ein Coccidiostatikum muß gute Wirksamkeit gegen verschiedene Coccidienarten in niedrigen Anwendungskonzentrationen, gute Verträglichkeit und eine daraus resultierende große therapeutische Breite aufweisen. Daneben sollten neue Coccidiostatika gegen bereits arzneiresistente Coccidienstämme wirksam sein.

Die Verbindungen der Formel I und ihre Salze zeigen bereits in sehr geringen Mengen einen ausgeprägten Effekt gegen verschiedene Erreger der Coccidiose beim Geflügel und anderen Tierspezies bei gleichzeitiger sehr guter Verträglichkeit. Darüber hinaus beeinflussen sie mehrfacharzneimittel-resistente Erreger der Coccidiose.

Die Verbindungen der Formel I können grundsätzlich als solche in Substanz verabreicht werden. Bevorzugt ist ihre Verwendung in Mischung mit geeignetem Trägermaterial. Die Salze sind insbesondere zur Anwendung in Trinkwasser geeignet.

Als Trägermaterial können die üblichen Futtermittelmischungen verwendet werden. Ein Wirkstoff der Formel I wird dabei dem Futter in einer Konzentration von 0,1 - 300 ppm, vorzugsweise 0,5 - 50 ppm, zugemischt. Im Vergleich zu den bekannten Aralkyl-Hexahydrotriazinen der EP 58 534, die nur in einem Bereich von 70 bis 200 ppm im Futter oder Trinkwasser wirksam sind, zeichnen sich die erfindungsgemäßen Verbindungen der Formel I und ihre Salze insbesondere durch ihre Wirksamkeit in den angegebenen niedrigen Konzentration bei gleichzeitig guter Verträglichkeit aus.

Ferner zeichnen sich die Verbindungen I durch hohe Stabilität insbesondere gegen Licht und Luft aus.

Alkalimetalle soll Li, Na, K, bevorzugt Na oder K bedeuten.

Erdalkalimetalle soll Ca oder Mg bedeuten.

Ammonium umfaßt neben $NH_4^+$ auch solche Verbindungen, in denen 1-4 Wasserstoffatome beispielsweise durch Alkyl-, Alkoxyalkyl-, Hydroxyalkyl-, Aminoalkyl, Phenylalkyl mit jeweils 1-24 C-Atomen im Alkylrest, oder Phenyl bedeuten. Es wurde gefunden, daß die Verbindungen I im Ames-Test keine mutagene Wirkung zeigen.

A: Chemische Beispiele

Beispiel 1 :
2-[3,5-Dichlor-4-(4-methylsulfonylphenoxy)phenyl]-1-methylhexahydro-1,2,4-triazin-3,5-dion

1 g 2-[3,5-Dichlor-4-(4-methylsulfonylphenoxy)phenyl]-hexahydro-1,2,4-triazin-3,5-dion wurde in 10 ml N-Methylpyrrolidon mit 10 ml Methyljodid vier Stunden und nach Zugabe von weiteren 10 ml Methyljodid nochmals drei Stunden auf 140 bis 160 °C erhitzt. Nach dem Abkühlen wurde mit Wasser versetzt, es schied sich ein langsam fest werdendes Öl ab, das aus Methanol unter Zusatz von Aktivkohle umkristallisiert wurde. Fp. 223 °C.
NMR-Spektrum, 270 MHz, DMSO-$d_6$, TMS als innerer Standard, δ-Werte in ppm: -$N^1$-$CH_3$ 2,7 (s) , -$N^1$-$CH_2$ 3,9 (s)
In analoger Verfahrensweise wurden jeweils aus den 2-substituierten Hexahydro-1,2,4-triazin-3,5-dionen durch Alkylierung die folgenden zusätzlich in 1-Position alkylierten Verbindungen der Formel I erhalten:

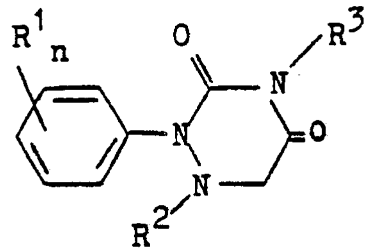

(I)

| Bsp. Nr. | $R^1$ | $R^2$ | $R^3$ | Fp. °C |
|---|---|---|---|---|
| 2 | $3,5-Cl_2$, $4-[4-CH_3S-C_6H_4O]-$ | $CH_3$ | H | 204 |
| 3 | $3,5-Cl_2$, $4-[4-CH_3SO-C_6H_4O]-$ | $CH_3$ | H | 228 |
| 4 | $3,5-Cl_2$, $4-[4-CH_3SO_2-C_6H_4O]-$ | Benzyl | H | 228 |
| 5 | $3,5-Cl_2$, $4-[4-Benzylthio-C_6H_4O]-$ | Benzyl | H | 178 |
| 6 | $3,5-Cl_2$, $4-[3-CH_3-4-\ CH_3S-C_6H_3O]-$ | $CH_3$ | H | 226 |
| 7 | $3,5-Cl_2$, $4-[4-Cl-C_6H_4S]-$ | $CH_3$ | H | 195 |
| 8 | 3-Cl, $4-[4-CH_3S-C_6H_4O]-$ | $CH_3$ | H | |
| 9 | $3,5-(CH_3)_2$, $4-[4-CH_3S-C_6H_4O]-$ | $CH_3$ | H | |
| 10 | $4-[3-CH_3-4-CH_3S-C_6H_3O]-$ | $CH_3$ | H | |
| 11 | $4-C_6H_4S$ | CH3 | H | 153 |

| Bsp. Nr. | $R^1$ | $R^2$ | $R^3$ | Fp.°C |
|---|---|---|---|---|
| 12 | $4\text{-}[4\text{-}CH_3S\text{-}C_6H_4S]\text{-}$ | $CH_3$ | H | |
| 13 | $3,5\text{-}Cl_2$, $4\text{-}[4\text{-}CH_3S\text{-}C_6H_4S]\text{-}$ | $CH_3$ | H | |
| 14 | $3,5\text{-}Cl_2$, $4\text{-}CH_3S$ | $CH_3$ | H | 266 |
| 15 | $3,5\text{-}(CF_3)_2$ | $CH_3$ | H | 143 |

Beispiel 16 : 1-Acetyl-2-[3,5-dichlor-4-(4-methylsulfonylphenoxy)phenyl]-hexahydro-1,2,4-triazin-3,5-dion

2 g 2-[3,5-Dichlor-4-(4-methylsulfonylphenoxy)phenyl]-hexahydro-1,2,4-triazin-3,5-dion wurden in 10 ml Acetanhydrid suspendiert. 1 ml konzentrierte Schwefelsäure wurde zugetropft, wobei sich die Suspension leicht erwärmte und das Ausgangsmaterial langsam in Lösung ging. Man rührte 2 bis 3 Stunden bei Zimmertemperatur nach und verrührte anschließend mit Wasser.
Das Produkt wurde abgesaugt, mit Wasser gewaschen und getrocknet und aus wenig Eisessig umkristallisiert, Fp. 267° C.

NMR-Spektrum, 60 MHz, DMSO-$d_6$, TMS als innerer Standard, $\delta$-Werte in ppm: $\text{-}N^1\text{-}COCH_3$ 2,1 (s), $\text{-}N^1\text{-}CH_2$ 4,6 (breites d, AB-Signal)

In analoger Verfahrensweise wurden jeweils aus den 2-substituierten Hexahydro-1,2,4-triazin-3,5-dionen durch Acylierung die folgenden zusätzlich in 1-Position acylierten Verbindungen der Formel I erhalten:

| Bsp. Nr. | $R^1$ | $R^2$ | $R^3$ | Fp.°C |
|---|---|---|---|---|
| 17 | $3,5\text{-}Cl_2$, $4\text{-}CH_3S\text{-}$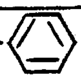$\text{-}O\text{-}$ | $CH_3CO$ | H | 255 |
| 18 | $3,5\text{-}Cl_2$, $4\text{-}CH_3SO_2\text{-}$ 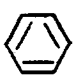 $\text{-}O\text{-}$ | $C_2H_5CO$ | H | 238 |
| 19 | $3,5\text{-}Cl_2$, $4\text{-}CH_3S\text{-}$ 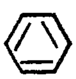 $\text{-}O\text{-}$ | $C_2H_5CO$ | H | 179 |
| 20 | $3,5\text{-}(CF_3)_2$ | $CH_3CO$ | H | 170 |
| 21 | $3,5\text{-}(CF_3)_2$ | $C_2H_5CO$ | H | |
| 21a | $3,5\text{-}Cl_2$, $4\text{-}[4\text{-}CH_3S\text{-}C_6H_4O]\text{-}$ | $C_6H_5CO$ | H | 217 |

Beispiel 22: 2-[3,5-Dichlor-4-(4-methylsulfonylphenoxy)-phenyl]-1-formyl-hexahydro-1,2,4-triazin-3,5-dion

1 g 2-[3,5-Dichlor-4-(4-methylsulfonylphenoxy)phenyl]-hexahydro-1,2,4-triazin-3,5-dion wurde in 10 ml Ameisensäure suspendiert. Nacheinander tropfte man 5 ml Propionylchlorid und 1 ml konzentrierte Schwefelsäure zu und rührte 5 Stunden bei 80° C. Man gab nach Abkühlen weitere 10 ml Propionylchlorid zur Vervollständigung der Reaktion hinzu und erhitzte unter Rühren nochmals 5 Stunden auf 80° C. Anschließend goß man auf Eiswasser, saugte die Fällung ab und kochte sie mit wenig Toluol aus. Der Rückstand wurde aus Isopropanol/Diisopropylether umkristallisiert, Fp. 212° C Zers..
NMR-Spektrum, 60 MHz, DMSO-$d_6$, TMS als innerer Standard, δ-Werte in ppm: -$N^1$-CHO 8, 7 (s), $N^1$-$CH_2$ 4,8 (s)

In analoger Weise erhielt man

Beispiel 23 : 2-[3,5-Dichlor-4-(4-methylthio-phenoxy)phenyl]-1-formyl-hexahydro-1,2,4-triazin-3,5-dion, Fp. 180° C Zers..

Beispiel 24 : 2-[3,5-Dichlor-4-(4-methylthiophenoxy)phenyl]-1,4-dimethyl-hexahydro-1,2,4-triazin-3,5-dion

0,7 g 2-[3,5-Dichlor-4-(4-methylthio-phenoxy)phenyl]-1-methyl-hexahydro-1,2,4-triazin-3,5-dion (Beispiel 2) wurden in 5 ml dimethylformamid kalt gelöst. Etwa 200 mg Natriumhydrid-Suspension (ca. 50%ig) wurden zugegeben, nach Beendigung der Gasentwicklung tropfte man 1 ml Methyljodid zu und rührte 4 Stunden bei Zimmertemperatur. Nach Ansäuern mit 2 n HCl wurde mit Methylenchlorid extrahiert, eingeengt und das zurückbleibende Öl mit Petrolether verrieben, wobei es kristallisierte; nach Umkristallisation aus Isopropanol Fp. 148° C.

NMR-Spektrum, 60 MHz, DMSO-$d_6$, TMS als innerer Standard,
δ-Werte in ppm: -$N^1$-$CH_3$ 2,7 (s) , -$N^4$-$CH_3$ 3,1 (s)
-$N^1$-$CH_2$ 4,0 (s)

In analoger Verfahrensweise wurden jeweils aus den 1,2-disubstituierten Hexahydro-1,2,4-triazin-3,5-dionen durch Alkylierung die folgenden zusätzlich in 4-Position alkylierten Verbindungen der Formel I erhalten:

| Bsp. Nr. | $R^1$ | $R^2$ | $R^3$ | Fp. °C |
|---|---|---|---|---|
| 25 | 3,5-$Cl_2$, 4-[4-$CH_3SO_2$-$C_6H_4O$]- | $CH_3$ | $CH_3$ | 178 |
| 26 | 3,5-$Cl_2$, 4-[4-$CH_3SO_2$-$C_6H_4O$]- | $CH_3$ | $C_2H_5$ | 181 |
| 27 | 3,5-$Cl_2$, 4-[4-$CH_3S$-$C_6H_4O$]- | $CH_3$ | $C_2H_5$ | |
| 28 | 3,5-$Cl_2$, 4-[4-$CH_3SO$-$C_6H_4O$]- | $CH_3$ | $CH_3$ | |
| 29 | 3,5-$Cl_2$, 4-[4-$CH_3SO$-$C_6H_4O$]- | $CH_3$ | $C_2H_5$ | |
| 30 | 3,5-$Cl_2$, 4-[4-$CH_3SO_2$-$C_6H_4O$]- | $CH_3$ | $C_4H_9$ | |
| 31 | 3,5-$Cl_2$, 4-[4-$CH_3SO_2$-$C_6H_4O$]- | $CH_3$ | Benzyl | 201 |
| 32 | 3,5-$Cl_2$, 4-[4-$CH_3SO_2$-$C_6H_4O$]- | Benzyl | Benzyl | |
| 33 | 3,5-$Cl_2$, 4-[3-$CH_3$-4-$CH_3S$-$C_6H_3O$]- | $CH_3$ | $CH_3$ | |

| Bsp. Nr. | $R^1$ | $R^2$ | $R^3$ | Fp. °C |
|---|---|---|---|---|
| 34 | 3,5-$Cl_2$, 4-[4-Cl-$C_6H_4S$]- | $CH_3$ | $CH_3$ | |
| 35 | 3,5-$(CH_3)_2$, 4-[4-$CH_3S$-$C_6H_4O$]- | $CH_3$ | $CH_3$ | |
| 36 | 3,5-$Cl_2$, 4-[4-$CH_3S$-$C_6H_4O$]- | $CH_3$ | $CH_3$ | |
| 37 | 3,5-$Cl_2$, 4-$CH_3S$- | $CH_3$ | $CH_3$ | |
| 38 | 3,5-$(CF_3)_2$ | $CH_3$ | $CH_3$ | 87 |
| 38a | 3,5-$(CF_3)_2$ | $CH_3$ | $C_2H_5$ | 91 |
| 38b | 3,5-$Cl_2$, 4-[4-$CH_3SO_2$-$C_6H_4O$]- | Benzyl | $CH_3$ | 185 |
| 38c | --------"---------- | $CH_3CO$ | $CH_3$ | 233 |
| 38d | 3,5-$(CF_3)_2$ | $CH_3$ | Benzyl | 108 |

Beispiel 39 : 2-[3,5-Dichlor-4-(4-methylsulfonylphenoxy)-phenyl]-4-methyl-hexahydro-1,2,4-triazin-3,5-dion

1 g 2-[3,5-Dichlor-4-(4-methylsulfonylphenoxy)phenyl]-hexahydro-1,2,4-triazin-3,5-dion wurde in 10 ml Dimethylformamid gelöst und unter Feuchtigkeitsausschluß mit ca. 250 mg Natriumhydrid-Suspension versetzt. Nach Beendigung der Gasentwicklung wurde 1 ml Methyljodid unter Rühren zugetropft und anschließend 5 h bei Zimmertemperatur weitergerührt. Die Reaktionslösung wurde auf Wasser gegossen, der Niederschlag abgesaugt und aus Isopropanol umkristallisiert, Fp. 217° C.

NMR-Spektrum, 60 MHz, DMSO-$d_6$, TMS als innerer Standard,
δ-Werte in ppm: -$N^1H$-$CH_2$ 6,5 (tr, J=8 Hz), -$N^4$-$CH_3$ 3,1 (s)
-$N^1H$-$CH_2$ 3,8 (d, J=8 Hz)

In analoger Verfahrensweise wurden jeweils aus den 2-substituierten Hexahydro-1,2,4-triazin-3,5-dionen durch Alkylierung die folgenden zusätzlich in 4-Position alkylierten Verbindungen der Formel I erhalten:

| Bsp. Nr. | $R^1$ | $R^2$ | $R^3$ | Fp.°C |
|---|---|---|---|---|
| 40 | 3,5-$Cl_2$, 4-[4-$CH_3S$-$C_6H_4O$]- | H | $CH_3$ | 163 |
| 41 | 3,5-$Cl_2$, 4-[4-$CH_3SO$-$C_6H_4O$ | H | $CH_3$ | |
| 42 | 3,5-$Cl_2$, 4-[4-$CH_3SO_2$-$C_6H_4O$]- | H | $C_2H_5$ | 237 |
| 43 | 3,5-$Cl_2$, 4-[4-$CH_3SO_2$-$C_6H_4O$]- | H | $C_4H_9$ | 256 |
| 44 | 3,5-$Cl_2$, 4-[4-$CH_3SO_2$-$C_6H_4O$]- | H | $CH_2$- 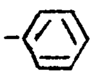 | 197 |
| 45 | 3,5-$Cl_2$, 4-[3-$CH_3$-4-$CH_3S$-$C_6H_3O$]- | H | $CH_3$ | |
| 46 | 3,5-$Cl_2$, 4-[4-Cl-$C_6H_4S$]- | H | $CH_3$ | |
| 47 | 3,5-$(CH_3)_2$, 4-[4-$CH_3S$-$C_6H_4O$]- | H | $CH_3$ | |
| 48 | 3,5-$Cl_2$, 4-[4-$CH_3S$-$C_6H_4S$]- | H | $CH_3$ | |
| 49 | 3,5-$Cl_2$, 4-$CH_3S$- | H | $CH_3$ | |
| 50 | 3,5-$(CF_3)_2$ | H | $CH_3$ | 98 |
| 50a | 3,5-$(CF_3)_2$ | H | $C_2H_5$ | 118 |
| 50b | ---"---- | H | $C_4H_9$ | 80 |
| 50c | ---"---- | H | Benzyl | 177 |

Darstellung der Verbindungen der Formel II:

Beispiel 51: 2-[3,5-Dichlor-4-(4-methylsulfonyl-phenoxy)phenyl]-hexahydro-1,2,4-triazin-3,5-dion

10 g 2-[3, 5-Dichlor-4-(methylsulfonyl-phenoxy)phenyl]-,2,4-triazin-3,5-(2H,4H)-dion wurden in 150 ml Eisessig heiß gelöst, mit 10 g Zinkstaub versetzt und zwei Stunden zum Rückfluß erhitzt. Anschließend wurde heiß filtriert und der zinkhaltige Rückstand dreimal mit 50 ml Eisessig ausgekocht. Die gesammelten Lösungen wurden unter vermindertem Druck eingeengt, mit Wasser versetzt und abgesaugt. Der verbleibende Feststoff wurde aus Methanol unter Zusatz von Aktivkohle umkristallisiert, Fp. 240° C Zers..

NMR-Spektrum, 60 MHz, DMSO-$d_6$, TMS als innerer Standard, δ-Werte in ppm: $-N^1\underline{H}-CH_2$ 6,53 (tr, J=8 Hz), $-N^1H-CH_2$ 3,7 (d, J=8 Hz), $CH_3SO_2-$ 3,2 (s)

Beispiel 52 : 2-[3,5-Dichlor-4-(4-methylthio-phenoxy)phenyl]-hexahydro-1,2,4-triazin-3,5-dion

20 g 2-[3,5-Dichlor-4-(4-methylthio-phenoxy)phenyl]-1,2,4-triazin-3,5-(2H,4H)-dion wurden in 200 ml Eisessig heiß gelöst. Man gab 25 g Zinn(II)chlorid ˋ 2 $H_2O$ und anschliessend 100 ml konzentrierte Salzsäure zu und erhitzte anschließend vier Stunden zum Rückfluß. Nach dem Abkühlen saugte man das Product ab, wusch mit Wasser neutral und kristallisierte aus 2-Methoxyethanol um, Fp. 239 °C.

NMR-Spektrum, 60 MHz, DMSO-$d_6$, TMS als innerer Standard, δ-Werte in ppm: $-N^1\underline{H}-CH_2$ 6,53 (tr, J=8 Hz), $-N^1H-CH_2$ 3,7 (d, J=8 Hz), $CH_3S-$ 2,45 (s)

B. Biologische Beispiele

Coccidiostatischer Effekt gegenüber Eimeria tenella

Küken (4-50 Tiere/Dosis) mit einem Körpergewicht von 35 bis 40 g wurden oral mittels Schlundsonde mit Eimeria tenella infiziert. Eine Infektionskontrolle (8 bis 50 Tiere) erhielt die gleiche Infektionsdosis wie die medikierten Küken (Dosisbereich 3,5 x $10^5$ sporulierte Oocysten pro Küken).

Die Testpräparate der Formel I wurden dem Futter, das die im Handel erhältliche übliche Zusammensetzung aufwies, in einem Mischer beigemischt. Dieses Futter wurde den Tieren während der Versuchsdauer ad libitum angeboten. In jedem Versuch wurde eine Nullkontrolle (d. h. 8 bis 50 nicht medikierte, nicht infizierte Tiere) mitgeführt. Die Versuchsdauer betrug ca. 10 Tage.

Bei Applikation der Verbindungen der Beispiele
1, 2, 3, 4, 5, 6, 7, 10, 11, 12, 14, 15, 17, 18, 19, 20, 20a, 22, 23, 24, 25, 26, 31, 38a, 38b, 38c, 38d, 39, 40, 42, 43, 44, 50, 50a, 50b, 50c
in einer Dosierung von 100 pm (bezogen auf das Futter) überlebten die medikierten Küken ohne jegliche Anzeichen einer Coccidiose; d.h. blutiger Kot, hohe Oocystenproduktion oder Darmläsionen wurden in diesen Fällen nicht beobachtet.

**Ansprüche**

1. Verbindungen der Formel I oder ihre Salze

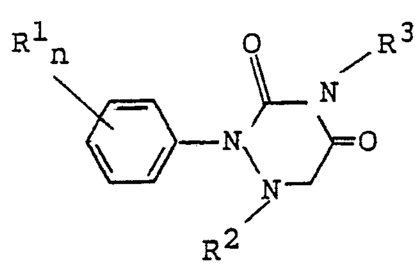

(I)

worin bedeuten
n
eins, zwei oder drei
und die einzelnen Substituenten
$R^1$
unabhängig voneinander
Phenoxy-, Phenylthio-, Phenylsulfinyl- oder Phenylsulfonylrest, der ein- oder zweifach durch F, Cl, Br, $CF_3$, Alkyl, Alkoxy, Alkylthio, Alkylsulfinyl oder Alkylsulfonyl mit jeweils 1 bis 6 C-Atomen im Alkylrest substituiert sein kann,
$R^2$
Wasserstoff, geradkettiges oder verzweigtes Alkyl oder Alkanoyl mit jeweils 1 bis 4 C-Atomen, Cycloalkyl mit 3 bis 7 C-Atomen, Benzyl oder Benzoyl, die unsubstituiert oder durch F, Cl, Br oder $(C_1-C_4)$-Alkyl substituiert sind;
$R^3$
Wasserstoff, geradkettiges oder verzweigtes Alkyl mit 1 bis 12 C-Atomen, Cycloalkyl mit 3 bis 7 C-Atomen oder unsubstituiertes oder durch F, Cl, Br oder $(C_1-C_4)$-Alkyl substituiertes Benzyl;
unter der Voraussetzung, daß mindestens einer der Reste $R^2$ und $R^3$ ungleich Wasserstoff ist,
sowie deren Alkali-, Erdalkali- oder Ammoniumsalze, sofern $R^3$ Wasserstoff bedeutet.

**2.** Verbindung I nach Anspruch 1, dadurch gekennzeichnet, daß bedeuten:
n
zwei oder drei; und die einzelnen Substituenten
$R^1$
unabhängig voneinander
Phenoxyrest, der ein- oder zweifach durch Cl, $CF_3$, $(C_1-C_4)$-Alkyl, $(C_1-C_4)$-Alkylthio, $(C_1-C_4)$-Alkylsulfinyl oder $(C_1-C_4)$-Alkylsulfonyl substituiert sein kann,
$R^2$ = $(C_1-C_4)$-Alkyl, $(C_1-C_4)$-Alkanoyl oder Benzyl;
$R^3$ = H, $(C_1-C_4)$-Alkyl oder Benzyl,
und worin vorzugsweise der Phenylrest in Formel I in 3,5-Stellung disubstituiert oder 3,4,5-Stellung trisubstituiert ist.

**3.** Verfahren zur Herstellung von substituierten Hexahydro-1,2,4-triazin-3,5-dionen der Formel I nach Anspruch 1, dadurch gekennzeichnet, daß man eine Verbindung der Formel II

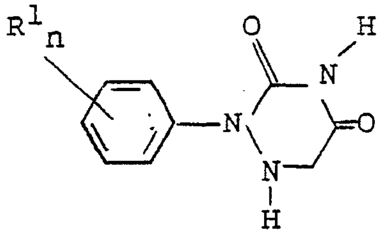

(II),

worin n und $R^1$ die zu Formel I angegebenen Bedeutungen haben, alkyliert oder acyliert.

**4.** Tierarzneimittel, dadurch gekennzeichnet, daß es eine Verbindung der Formel I nach Anspruch 1 enthält oder aus ihr besteht.

**5.** Verwendung einer Verbindung der Formel I nach Anspruch 1 zur Bekämpfung von Protozoenerkrankungen, insbesondere von Coccidiose.

Patentansprüche für folgenden Vertragsstaat : AT

**1.** Verfahren zur Herstellung von Verbindungen der Formel I

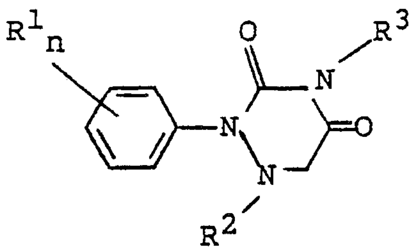

(I)

worin bedeuten
n
eins, zwei oder drei
und die einzelnen Substituenten
$R^1$
unabhängig voneinander
Phenoxy-, Phenylthio-, Phenylsulfinyl- oder Phenylsulfonylrest, der ein- oder zweifach durch F, Cl, Br, $CF_3$, Alkyl, Alkoxy, Alkylthio, Alkylsulfinyl oder Alkylsulfonyl mit jeweils 1 bis 6 C-Atomen im Alkylrest substituiert sein kann,
$R^2$
Wasserstoff, geradkettiges oder verzweigtes Alkyl oder Alkanoyl mit jeweils 1 bis 4 C-Atomen, Cycloalkyl mit 3 bis 7 C-Atomen, Benzyl oder Benzoyl, die unsubstituiert oder durch F, Cl, Br oder ($C_1$-$C_4$)-Alkyl substituiert sind;
$R^3$
Wasserstoff, geradkettiges oder verzweigtes Alkyl mit 1 bis 12 C-Atomen, Cycloalkyl mit 3 bis 7 C-Atomen oder unsubstituiertes oder durch F, Cl, Br oder ($C_1$-$C_4$)-Alkyl substituiertes Benzyl;
unter der Voraussetzung, daß mindestens einer der Reste $R^2$ und $R^3$ ungleich Wasserstoff ist, dadurch gekennzeichnet, daß man eine Verbindung der Formel II

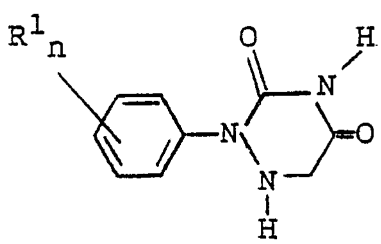

(II),

worin n und $R^1$ die zu Formel I angegebene Bedeutung haben, alkyliert oder acyliert.

2. Verfahren gemäß Anspruch 1, dadurch gekennzeichnet, daß in Formel I bedeuten:
n
zwei oder drei; und die einzelnen Substituenten
$R^1$
unabhängig voneinander
Phenoxyrest, der ein- oder zweifach durch Cl, $CF_3$, ($C_1$-$C_4$)-Alkyl, ($C_1$-$C_4$)-Alkylthio, ($C_1$-$C_4$)-Alkylsulfinyl oder ($C_1$-$C_4$)-Alkylsulfonyl substituiert sein kann,
$R^2$ = ($C_1$-$C_4$)-Alkyl, ($C_1$-$C_4$)-Alkanoyl oder Benzyl;
$R^3$ = H, ($C_1$-$C_4$)-Alkyl oder Benzyl,
und worin vorzugsweise der Phenylrest in Formel I in 3,5-Stellung disubstituiert oder 3,4,5-Stellung trisubstituiert ist.

3. Tierarzneimittel, dadurch gekennzeichnet, daß es eine Verbindung der Formel I nach Anspruch 1 enthält oder aus ihr besteht.

4. Verwendung einer Verbindung der Formel I nach Anspruch 1 zur Bekämpfung von Protozoenerkrankungen, insbesondere von Coccidiose.

## Claims

1. A compound of the formula I or the salts thereof

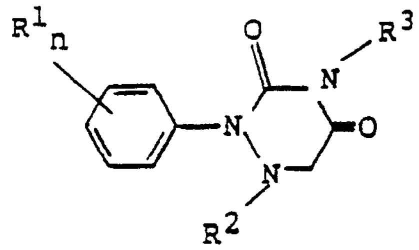

(I)

in which:
n
denotes one, two or three,
and the individual substituents
$R^1$,
independently of one another, denote phenoxy, phenylthio, phenylsulfinyl or phenylsulfonyl radical which can be substituted once or twice by F, Cl, Br, $CF_3$, alkyl, alkoxy, alkylthio, alkylsulfinyl or alkylsulfonyl, each having 1 to 6 carbon atoms in the alkyl radical,
$R^2$
denotes hydrogen, straight-chain or branched alkyl or alkanoyl each having 1 to 4 carbon atoms, cycloalkyl having 3 to 7 carbon atoms, benzyl or benzoyl each of which is unsubstituted or substituted by F, C1, Br or $(C_1-C_4)$-alkyl;
$R^3$
denotes hydrogen, straight-chain or branched alkyl having 1 to 12 carbon atoms, cycloalkyl having 3 to 7 carbon atoms, or benzyl which is unsubstituted or substituted by F, Cl, Br or $(C_1-C_4)$-alkyl;
with the proviso that at least one of the radicals $R^2$ and $R^3$ is not hydrogen,
and their alkali metal, alkaline earth metal or ammonium salts when $R^3$ denotes hydrogen.

2. A compound I as claimed in claim 1, wherein
n
denotes two or three; and the individual substituents
$R^1$,
independently of one another, denote phenoxy radical which can be substituted once or twice by Cl, $CF_3$, $(C_1-C_4)$-alkyl, $(C_1-C_4)$-alkylthio, $(C_1-C_4)$-alkylsulfinyl or $(C_1-C_4)$-alkylsulfonyl,
$R^2$ = $(C_1-C_4)$-alkyl, $(C_1-C_4)$-alkanoyl or benzyl,
$R^3$ = H, $(C_1-C_4)$-alkyl or benzyl,
and in which preferably the phenyl radical in formula I is disubstituted in the 3,5-position or trisubstituted in the 3,4,5-position.

3. A process for the preparation of substitutedhexahydro-1,2,4-triazine-3,5-diones of the formula I as claimed in claim 1, which comprises alkylation or acylation of a compound of the formula II

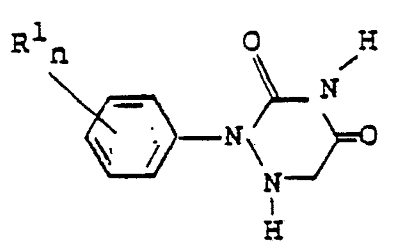

(II)

in which n and $R^1$ have the meanings indicated for formula I.

4. A veterinary medicament which contains or is composed of a compound of the formula I as claimed in claim 1.

5. The use of a compound of the formula I as claimed in claim 1 for the control of protozoal diseases, in particular coccidiosis.

Claims for the following Contracting State : AT

1. A process for the preparation of a compound of the formula I

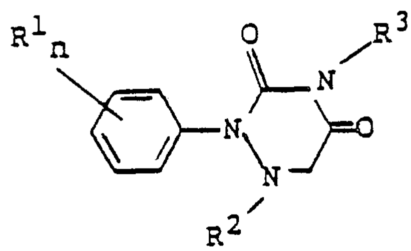

(I)

in which:

n

denotes one, two or three; and the individual substituents

$R^1$,

independently of one another, denote phenoxy, phenylthio, phenylsulfinyl or phenylsulfonyl radical which can be substituted once or twice by F, Cl, Br, $CF_3$, alkyl, alkoxy, alkylthio, alkylsulfinyl or alkylsulfonyl, each having 1 to 6 carbon atoms in the alkyl radical,

$R^2$

denotes hydrogen, straight-chain or branched alkyl or alkanoyl each having 1 to 4 carbon atoms, cycloalkyl having 3 to 7 carbon atoms, benzyl or benzoyl each of which is unsubstituted or substituted by F, Cl, Br or $(C_1-C_4)$-alkyl;

$R^3$

denotes hydrogen, straight-chain or branched alkyl having 1 to 12 carbon atoms, cycloalkyl having 3 to 7 carbon atoms, or benzyl which is unsubstituted or substituted by F, Cl Br or $(C_1-C_4)$-alkyl;

with the proviso that at least one of the radicals $R^2$ and $R^3$ is not hydrogen, which comprises alkylation or acylation of a compound of the formula II

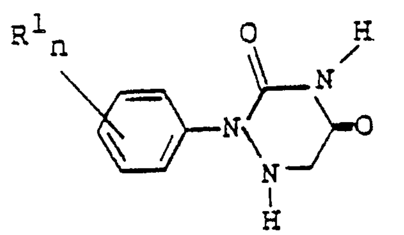

(II)

in which n and $R^1$ have the meaning indicated for formula I.

2. The process as claimed in claim 1, wherein in formula I

n

denotes two or three; and the individual substituents

$R^1$,

independently of one another, denote phenoxy radical which can be substituted once or twice by Cl, $CF_3$, $(c_1-c_4)$-alkyl, $(C_1-C_4)$-alkylthio, $(C_1-C_4)$-alkylsulfinyl or $(C_1-C_4)$-alkylsulfonyl,

$R^2$ = $(C_1-C_4)$-alkyl, $(C_1-C_4)$-alkanoyl or benzyl,

$R^3$ = H, $(C_1-C_4)$-alkyl or benzyl,

and in which preferably the phenyl radical in formula I is disubstituted in the 3,5-position or trisubstituted in the 3,4,5-position.

3. A veterinary medicament which contains or is composed of a compound of the formula I as claimed in claim 1.

4. The use of a compound of the formula I as claimed in claim 1 for the control of protozoal diseases, in particular coccidiosis.

## Revendications

1. Composés de formule I ou leurs sels :

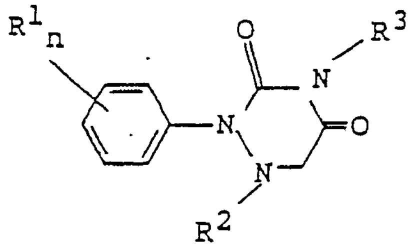

(I)

dans laquelle :
n
est égal à 1, a 2 ou à 3,
les $R^1$
représentent chacun, indépendamment l'un de l'autre, un radical phénoxy, phénylthio, phénylsulfinyle ou phénylsulfonyle qui peut porter un ou deux substituants pris dans l'ensemble constitué par F, Cl, Br, $CF_3$ et les radicaux alkyles, alcoxy, alkylthio, alkylsulfinyles et alkylsulfonyles contenant chacun de 1 à 6 atomes de carbone dans la partie alkyle,
$R^2$
représente l'hydrogène, un radical alkyle ou alcanoyle, linéaire ou ramifié, qui contient de 1 à 4 atomes de carbone, un cycloalkyle contenant de 3 à 7 atomes de carbone, ou un radical benzyle ou benzoyle non substitué ou porteur de F, de Cl, de Br ou d'un alkyle en $C_1$-$C_4$, et
$R^3$
représente l' hydrogène, un alkyle en $C_1$-$C_{12}$, linéaire ou ramifié, un cycloalkyle en $C_3$-$C_7$, ou un benzyle non substitué ou porteur de F, de Cl, de Br ou d'un alkyle en $C_1$-$C_4$,
avec la condition qu'au moins un des symboles $R^2$ et $R^3$ ne soit pas l'hydrogène,
ainsi que leurs sels de métaux alcalins, de métaux alcalinoterreux et d'ammoniums dans la mesure où $R^3$ représente l'hydrogène.

2. Composés selon la revendication 1, caractérisés en ce que :
n
est égal à 2 ou à 3,
les $R^1$
représentent chacun, Indépendamment l'un de l'autre, un radical phénoxy qui peut porter un ou deux substituants pris dans l'ensemble constitué par Cl, $CF_3$, les alkyles en $C_1$-$C_4$, les alkylthio en $C_1$-$C_4$, les alkylsulfinyles en $C_1$-$C_4$ et les alkylsulfonyles en $C_1$-$C_4$,
$R^2$
représente un alkyle en $C_1$-$C_4$, un alcanoyle en $C_1$-$C_4$ ou un benzyle, et
$R^3$
représente H, un alkyle en $C_1$-$C_4$ ou un benzyle, et dans lesquels le radical phényle de la formule I est de préférence disubstitué en position 3,5 ou trisubstitué en position 3,4,5.

3. Procédé pour préparer des hexahydrotriazine-1,2-4 diones-3,5 substituées de formule I selon la revendication 1, procédé caractérisé en ce qu'on alkyle ou acyle un composé répondant à la formule II :

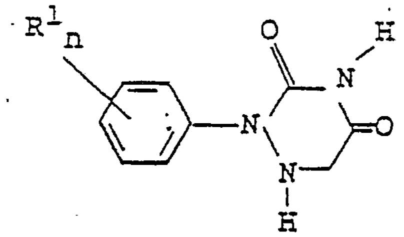

(II)

dans laquelle n et $R^1$ ont les significations qui leur ont été données à propos de la formule I.

**4.** Médicament vétérinaire caractérisé en ce qu'il contient un composé de formule I selon la revendication 1 ou est constitué d'un tel composé.

**5.** Application d'un composé de formule I selon la revendication 1 pour la lutte contre des maladies à protozoaires, plus particulièrement contre la coccidiose.

Revendications pour l,Etat contractant suivant : AT

**1.** Procédé pour préparer des composés répondant à la formule I :

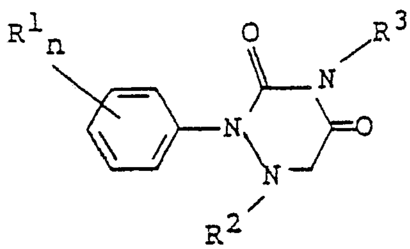

(I)

dans laquelle :

n

est égal à 1, à 2 où à 3,

les $R^1$

représentent chacun, indépendamment l'un de l'autre, un radical phénoxy, phénylthio, phénylsulfinyle ou phénylsulfonyle qui peut porter un ou deux substituants pris dans l'ensemble constitué par F, Cl, Br, $CF_3$ et les radicaux alkyles, alcoxy, alkylthio, alkylsulfinyles et alkylsulfonyles contenant chacun de 1 à 6 atomes de carbone dans la partie alkyle,

$R^2$

représente l'hydrogène, un radical alkyle ou alcanoyle, linéaire ou ramifié, qui contient de 1 à 4 atomes de carbone, un cycloalkyle contenant de 3 à 7 atomes de carbone, ou un radical benzyle ou benzoyle non substitué ou porteur de F, de Cl, de Br ou d'un alkyle en $C_1$-$C_4$, et

$R^3$

représente l'hydrogène, un alkyle en $C_1$-$C_{12}$, linéaire ou ramifié, un cycloalkyle en $C_3$-$C_7$, ou un benzyle non substitué ou porteur de F, de Cl, de Br ou d'un alkyle en $C_1$-$C_4$,

avec la condition qu'au moins un des symboles $R^2$ et $R^3$ ne soit pas l'hydrogène,

procédé caractérisé en ce qu'on alkyle ou acyle un composé répondant à la formule II :

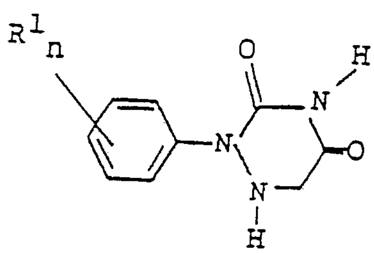

(II)

dans laquelle n et $R^1$ ont les significations qui leur ont été données à propos de la formule I.

2. Procédé selon la revendication 1 caractérisé en ce que, dans la formule I :
n
est égal à 2 ou à 3,
les $R^1$
représentent chacun, indépendamment l'un de l'autre, un radical phénoxy qui peut porter un ou deux substituants pris dans l' ensemble constitué par Cl, $CF_3$, les alkyles en $C_1$-$C_4$, les alkylthio en $C_1$-$C_4$, les alkylsulfinyles en $C_1$-$C_4$ et les alkylsulfonyles en $C_1$-$C_4$,
$R^2$
représente un alkyle en $C_1$-$C_4$, un alcanoyle en $C_1$-$C_4$ ou un benzyle, et
$R^3$
représente H, un alkyle en $C_1$-$C_4$ ou un benzyle,
et le radical phényle de la formule I est de préférence disubstitué en position 3,5 ou trisubstitué en position 3,4,5.

3. Médicament vétérinaire caractérisé en ce qu'il contient un composé de formule I selon la revendication 1 ou est constitué d'un tel composé.

4. Application d'un composé de formule I selon la revendication 1 pour la lutte contre des maladies à protozoaires, plus particulièrement contre la coccidiose.